Europäisches Patentamt

European Patent Office

Office européen des brevets

(11) Publication number : **0 631 722 A1**

(12) **EUROPEAN PATENT APPLICATION**

(21) Application number : **94401399.4**

(51) Int. Cl.$^6$ : **A01N 25/30, A61L 2/16**

(22) Date of filing : **22.06.94**

(30) Priority : **28.06.93 JP 157516/93**

(43) Date of publication of application :
**04.01.95 Bulletin 95/01**

(84) Designated Contracting States :
**BE DE FR GB NL**

(71) Applicant : **Hishida, Iwao**
**4-16 Hattori Minami-machi 4-chome**
**Toyonaka-shi, Osaka 561 (JP)**

(72) Inventor : **Hishida, Iwao**
**4-16 Hattori Minami-machi 4-chome**
**Toyonaka-shi, Osaka 561 (JP)**

(74) Representative : **Kedinger, Jean-Paul et al**
**c/o Cabinet Malemont**
**42, avenue du Président Wilson**
**F-75116 Paris (FR)**

(54) **Method for exterminating microbes for disinfection and deodorization.**

(57) Disclosed is a method for exterminating microbes for disinfection and deodorization by the use of a microbicidal composition containing, as the active ingredient, at least one nitrogen-containing nonionic surfactant preferably selected from polyoxyethylene fatty acid amides and polyoxyethylene alkylamines. Preferably used are polyoxyethylene fatty acid amides where the fatty acid amide moiety has from 12 to 24 carbon atoms and the number of mols of ethylene oxide added to the moiety is 20 or less, and polyoxyethylene alkylamines where the higher alkylamine moiety has from 12 to 24 carbon atoms and the number of mols of ethylene oxide added to the moiety is 20 or less. The microbicidal composition is highly effective against both gram-negative microbes and gram-positive microbes including MRSA, while having an excellent disinfecting and deodorizing activity.

EP 0 631 722 A1

## Background of the Invention

### Field of the Invention

The present invention relates to a method for exterminating microbes for disinfection and deodorization applicable to various fields of food-related environments such as dining halls, kitchens, etc., kitchen utensils, food-wrapping materials such as food containers, food wrapping films, etc., medical service-related environments such as passageways in hospitals, floors, walls and curtains in sickrooms, etc., gloves and gowns for doctors and nurses, medical appliances, medical supplies, etc.

### Description of the Related Art

As means for exterminating microbes for disinfection and deodorization in food-related fields, for example, there have heretofore been known various microbicide-containing plastic products, such as food containers, food-wrapping films, films for maintaining the freshness of vegetables and fruits, chopping boards, etc. These are to prevent the growth of bacteria that cause sitotoxism, such as Escherichia coli, Staphylococcus aureus, Streptococcus pyogenes, Pseudomonas aeruginosa, etc., and also to exterminate putrefying bacteria that cause offensive odors. The requirements for microbicides to be used as additives to the above-mentioned food-wrapping plastics are such that (1) they have low toxicity, (2) their microbicidal activity lasts for a long period of time to several years and, additionally in Japan, (3) they pass the Food Sanitation Standard of Notification No. 370 of the Welfare Ministry of Japan, (4) they are approved by the Polyolefin Sanitation Conference, etc. Up to this time, however, there are known no organic compounds that meet the above-mentioned requirements and have been approved usable as additives to food-wrapping plastics. On the other hand, as inorganic compounds, silver ion compounds such as typically silver nitrate have low toxicity and therefore have been partly used as additives to plastics. However, even though they have low toxicity, silver itself is a toxic substance. In addition, regarding their microbicidal activity, they are effective against gram-negative microbes such as Escherichia coli, etc. but are poorly effective or are rather quite ineffective against gram-positive microbes such as methicillin-resistant Staphylococcus aureus (MRSA), etc. Moreover, since silver compounds are inorganic compounds, they are unevenly localized only in the inside of shaped plastics, if added thereto, so that they cannot be exposed out on the surfaces of shaped products and therefore their microbicidal activity is difficult to effectively display. Furthermore, since silver compounds start from silver nitrate, they are noticeably discolored when exposed to heat or ultraviolet rays. Therefore, they are not suitable as additives to plastics. In addition, since silver ion compounds exterminate microbes due to the force of their silver ions, they are quite ineffective in the absence of water.

For the purpose of exterminating bacteria such as hospital infectious bacteria MRSA which grow in hospitals and which have raised a grave nationwide issue in these days via newspapers, television, etc., as well as Mycobacterium tuberculosis, Escherichia coli, Pseudomonas aeruginosa, etc. and of preventing the growth of such bacteria, it is desired that disposable gloves and gowns for doctors and nurses and also polyolefin bags and films for wrapping instruments shall also have a microbicidal activity. In particular, due to MRSA, it has become extremely important to disinfect hospital-related environments and to exterminate microbes in them, which has now turned into a grave problem to be solved urgently. However, there are known no films containing effective microbicides at present.

The most popular disinfectants used at present are alcohols. In addition to this, mentioned are cationic surfactants which are referred to as cationic detergents as well as TEGO invert soap (made by Gold Schmidt A.G.) of ampholytic surfactants. However, alcohols cannot be kneaded into plastics, as they have a boiling point of from 60 to 80°C. In addition, the microbicidal activity of alcohols greatly varies, depending on their concentrations and the kinds of bacteria which are to be exterminated by them. For instance, alcohols have a poor microbicidal activity against cocci of Staphylococcus so that such cocci may live for a long period of time even though alcohols are applied thereto. Staphylococcus aureus and Escherichia coli may live for about one hour or more in the presence of 60 % alcohol which is generally used as a disinfectant. In addition, alcohols have a high vaporization speed and their microbicidal activity does not last long. Moreover, alcohols have still another drawback in that they roughen the skins of human bodies.

On the other hand, cationic surfactants and ampholytic surfactants are both soluble in water or hydrophilic so that they are not suitable for oleophilic plastics. It is said that additives to general thermoplastics are needed generally to have heat resistance to about 240°C in order that they are resistant to temperatures at which thermoplastics are shaped or machined. However, the heat resistance of cationic surfactants and ampholytic surfactants such as those mentioned above is limited to about 200°C so that they are difficult to use as additives to plastics.

Quaternary ammonium salt type cationic surfactants, such as benzalkonium and benzalkonium chloride, have low toxicity and are odorless while having an excellent microbicidal activity and they are much used as microbicides in these days. However, the fatal defects in such quaternary ammonium salt type cationic surfactant are such that they completely lose their microbicidal activity in the presence of anionic substances such as synthetic detergents and that their microbicidal activity is greatly lowered in the presence of proteins. In addition, when quaternary ammonium salt type cationic surfactants are in blood, they display a hemolytic effect. Therefore, care should be taken in using them. Moreover, these cationic surfactants are effective against gram-positive microbes but are poorly effective against gram-negative microbes such as Escherichia coli, Pseudomonas aeruginosa, etc.

TEGO type ampholytic surfactants also have a strong microbicidal activity and they are applied to meat-related and food-related materials and are used for washing hands. It is said that their activity is not lowered even in the presence of salts, lime, proteins or blood sugar. However, these ampholytic surfactants are defective in that they are extremely bitter even though diluted so that they are unsuitable for sprays, that their activity noticeably varies depending on the ambient pH condition, that they are poorly effective against gram-negative microbes and that they are ineffective against putrefactive microbes and paradoxical bacilli or, that is, they are unsuitable for deodorants or for prevention of putrefaction of water.

As mentioned above, no conventional microbicides are satisfactory with respect to their microbicidal, disinfecting and deodorizing activities.

Summary of the Invention

I, the present inventor earnestly studied in consideration of the above-mentioned various problems in conventional microbicides and, as a result, has found that nitrogen-containing nonionic surfactants have an excellent microbicidal activity and are resistant to temperatures at which plastics are shaped or worked and that they may be widely modified into water-soluble or oleophilic forms by suitably selecting the constitutive elements. On the basis of these finding, I have completed the present invention.

Specifically, the present invention provides a method for exterminating microbes by applying a composition containing, as the active ingredient, a nitrogen-containing nonionic surfactant thereto. The method is effective for disinfection and deodorization and also for preventing putrefaction of water.

Examples of the nitrogen-containing nonionic surfactant include polyoxyethylene fatty acid amides, polyoxyethylene alkylamines, alkylamine oxides and fatty acid alkanolamides. Of these, preferred are polyoxyethylene fatty acid amides and polyoxyethylene alkylamines. Especially preferred are polyoxyethylene fatty acid amides where the fatty acid amide moiety has from 12 to 24 carbon atoms and the number of mols of ethylene oxide added to the moiety is 20 or less, and polyoxyethylene alkylamines where the higher alkylamine moiety has from 12 to 24 carbon atoms and the number of mols of ethylene oxide added to the moiety is 20 or less.

One preferred embodiment of the present invention is to apply a solution composition or an emulsion composition containing, as the active ingredient, the above-mentioned nitrogen-containing nonionic surfactant to the objects to be disinfected or sterilized by the composition. The embodiment includes a method of directly sprinkling or spraying the solution composition or the emulsion composition containing, as the active ingredient, the above-mentioned nitrogen-containing nonionic surfactant to the objects to be disinfected or sterilized by the composition; a method of applying the solution composition or the emulsion composition containing, as the active ingredient, the above-mentioned nitrogen-containing nonionic surfactants to the objects to be disinfected or sterilized by the composition by dipping the objects, such as fingers, eating utensils, medical instruments, etc., in the composition; a method of applying the solution composition or the emulsion composition containing, as the active ingredient, the above-mentioned nitrogen-containing nonionic surfactant to mops or dusters by spraying or dipping, followed by wiping eating utensils, medical instruments, etc, with the mops or dusters or wiping and cleaning floors, walls, furniture, etc. with them; and a method of applying the solution composition or the emulsion composition containing, as the active ingredient, the above-mentioned nitrogen-containing nonionic surfactant to sanitary materials or sanitary articles by spraying or dipping.

As other embodiments of the present invention, mentioned are a method of applying a wax containing, as the active ingredient, the above-mentioned nitrogen-containing nonionic surfactant kneaded thereinto to floors; a method of putting oils and fats or waxes which are solid at a normal temperature or crystalline resin containing, as the active ingredient, the above-mentioned nitrogen-containing nonionic surfactant kneaded thereinto in a liquid to be disinfected or sterilized with the oils and fats or the wax or the resin; and a method of shaping a thermoplastic resin containing, as the active ingredient, the above-mentioned nitrogen-containing nonionic surfactant into films, fibers and other shaped articles.

The sterilizing method of the present invention, using nitrogen-containing nonionic surfactants, is extremely effective in exterminating any microbes of gram-negative Escherichia coli and gram-positive Staphylococcus

3

aureus which are microbes causing sitotoxism and is additionally effective in rapidly exterminating microbes of MRSA. Therefore, the method is effective for disinfection. In addition, the method is also effective for deodorization as putrefactive microbes are completely exterminated by the method. Hardly water-soluble nitrogen-containing nonionic surfactants added to thermoplastics by kneading them hardly dissolve out from the surfaces of shaped plastic products made of them and therefore they may maintain their microbicidal activity for a long period of time. In addition, such nitrogen-containing nonionic surfactants have a boiling point of 250°C or higher and are therefore sufficiently resistant to temperatures at which thermoplastics are shaped and worked. These nitrogen-containing nonionic surfactants have been approved for additives to plastics for food-packaging containers as dispersing agents for pigments and antistatic agents and have passed the Food Sanitation Standard of Notification No. 370 of the Welfare Ministry of Japan. They have been verified to have low toxicity. Therefore, the industrial value of the sterilizing method of the present invention is extremely high.

Detailed Description of the Invention

Of the nitrogen-containing nonionic surfactants for use in the present invention, polyoxyethylene fatty acid amides (higher fatty acid amide condensates) are polymers to be formed by addition polymerization of ethylene oxides to higher fatty acid acids, or dehydrated condensates to be formed by dehydrating condensation of higher fatty acid amides and polyethylene glycols. These are produced by adding ethylene oxides to two active hydrogens bonded to the nitrogen in higher fatty acid amides by addition polymerization, or by adding polyethylene glycols to the same by dehydrating condensation. They are nonionic surfactants having cationic properties. Polyoxyethylene alkylamines (higher alkylamine condensates) are weakly-cationic, nonionic surfactants to be obtained by condensation of higher alkylamines having two active hydrogens bonded to the nitrogen therein and ethylene oxides. Thus, polyoxyethylene fatty acid amides and polyoxyethylene alkylamines have properties of weakly-cationic surfactants, being somewhat different from ordinary nonionic surfactants. Of these, condensates in which the number of carbon atoms constituting the higher fatty acid amide moiety or the higher alkylamine moiety is from 12 to 24 and the number of mols of ethylene oxide added to the moiety is 20 or less have the above-mentioned cationic characteristics. In particular, those in which the number of mols of ethylene oxide added to the above-mentioned moiety is smaller have more cationic characteristics. Naturally, therefore, condensates in which the number of carbon atoms constituting the higher fatty acid amide moiety or the higher alkylamine moiety is from 12 to 24 and the number of mols of ethylene oxide added to the moiety is 20 or less are preferably employed in the present invention. In particular, those in which the number of carbon atoms constituting the higher fatty acid amide moiety or the higher alkylamine moiety is from 12 to 18 and the number of mols of ethylene oxide added to the moiety is 10 or less are more preferred. These polyoxyethylene fatty acid amides and polyoxyethylene alkylamines are characterized in that both water-soluble ones and ones which are hardly soluble in water but are soluble in alcohols, hydrocarbons and other organic solvents may freely be produced by employing suitable production methods, especially by suitably selecting and adjusting the kinds of higher fatty acid amides and those of higher alkylamines to be reacted and the number of mols of ethylene oxide to be added thereto. Needless-to-say, hardly water-soluble ones are preferably used when added to plastics by kneading.

The amount of the nitrogen-containing nonionic surfactant to be used in the method of the present invention is not critical but may suitably be determined according to the object of using it. Where it is added to a thermoplastic by kneading and the resulting thermoplastic mixture is shaped into a shaped article, the amount of the surfactant is preferably at least 0.1 % by weight relative to the thermoplastic. In the other cases, where it is used as a liquid for spray, or it is formulated into a microbicidal solution or emulsion, or it is used as a cleaning liquid for wiping, or it is applied to mops or dusters by dipping, or it is added to oils and fats or waxes which are solid at a normal temperature by kneading and the resulting mixture is solidified to solid pellets, the amount of the surfactant is preferably at least 0.05 % by weight.

As above-mentioned oils and fats or waxes which are solid at a normal temperature, there are natural waxes and synthetic waxes. The examples of natural waxes are paraffin wax, petroleum wax such as microcrystalline wax, etc., vegetable wax such as carnauba wax, candelilla wax, Japan wax, etc., animal wax such as bee wax, chinese wax, spermaceti wax, etc., and mineral wax such as montan wax, ozocerite wax etc. As the synthetic waxes, there are mentioned alcohol, acid, ester, ketone and amine which are all aliphatic, chrorinated naphthalene, synthetic mineral wax, synthetic animal wax. The typical example of the synthetic waxes are carbowaxes, hoechst waxes, polyethylene, etc.

As thermoplastic resins to which the surfactant of the present invention is added by kneading, for example, mentioned are polyethylenes, polypropylenes, polyvinyl chlorides, polystyrenes, polyamides (nylons), polyesters, etc. Considering the balance of their properties and costs, polyethylenes and polypropylenes are especially preferred.

4

According to the present invention, the above-mentioned nitrogen-containing nonionic surfactants may be combined with alcoholic microbicides, cationic surfactants, ampholytic surfactants, microbicides containing metal ions such as copper, zinc or silver ions, anti-fungal agents, etc.

Various embodiments and uses of the present invention are illustrated below, in which % and parts are by weight.

(1) A liquid prepared by dissolving the above-mentioned nitrogen-containing nonionic surfactant in an alcohol or solvent (for example, refer to Examples 3 and 4 mentioned hereinafter) is formulated into an aerosol or is mixed with about 30 % of water. This is directly used by sprinkling or spraying. For example, this is applied to sickrooms, operating rooms, white coats, curtains, etc. so as to sterilize or disinfect them, or to garbage or shoes so as to sterilize or deodorize them.

(2) Mops or dusters are dipped in an emulsion prepared by adding the above-mentioned nitrogen-containing nonionic surfactant to a mixture comprising a water-miscible solvent, such as ethanol, and water followed by emulsifying them (for example, refer to Example 5 mentioned hereinafter). These mops and dusters are used for wiping and cleaning floors, passageways, desks, doors, etc. The emulsion may also be used as a surface treating agent for treating clothes, curtains, sheets, etc.

(3) Mops or dusters are dipped in a solution prepared by dissolving the above-mentioned nitrogen-containing nonionic surfactant in a mineral oil, for example a solution comprised of 10 % of a nitrogen-containing nonionic surfactant as the microbicide, 80 % of liquid paraffin oil and 10 % of xylene, and these are used for wiping and cleaning floors, passageways, etc. If door mats (made of cotton, polyesters, rayons or their mixtures) are dipped in the solution, sterilized and disinfected mats are obtained.

(4) When a floor wax containing the following nitrogen-containing nonionic surfactant and having the composition mentioned below is applied to floors or passageways made of marble, wood or plastics once a month or so, the floors and passageways are sufficiently sterilized and disinfected.

| | |
|---|---|
| (i) Emulsion of nitrogen-containing nonionic surfactant (NV 20 %; made by Tohwa Chemical Co.) | 1.5 parts |
| (ii) Acrylic emulsion (NV 40 %; made by Rohm & Haas Co.) | 47 parts |
| Water | 23 parts |
| (mixed for 2 minutes) | |
| (iii) Filming aid (ethylene diglycol; made by Daisel Chemical Co.) | 4.5 parts |
| Water | 8.5 parts |
| (mixed for 10 minutes) | |
| (iv) Polyethylene wax emulsion (NV 40 %; made by Toho Chemical Co.) | 10 parts |
| (v) Defoaming agent (Antifoam; made by Sumitomo 3 M's Co.) | 0.2 parts |

(5) Solid pellets prepared by kneading the above-mentioned nitrogen-containing nonionic surfactant and oils and fats or waxes which are solid at a normal temperature or crystalline resin followed by solidifying the resulting mixture (for example, refer to Example 6 mentioned hereinafter) may be used for preventing putrefaction of water. For example, when they are dipped in water. they are effective for 6 months to one year. In the case, they are also effective in prolonging the life of flowers. In addition, they may also be used for preventing putrefaction of machine oils.

(6) The above-mentioned higher alkylamine condensate is kneaded with a thermoplastic resin (nylon, polyethylene, polypropylene, etc.) and shaped into films, fibers and other shaped articles (for example, refer to Examples 1 and 2 mentioned hereinafter). In this way, for example, produced are food-wrapping films, other packaging materials, gloves, aprons, tablecloths, chopping boards, containers, sanitary articles such as toothbrushes, non-woven fabrics, etc.

(7) Paper, or fibers (or fabrics) of cotton, polyesters, rayons, polyamides, polyethylenes, polypropylene-ethylenes or vinyl acetate copolymers dipped in any of a liquid obtained by dissolving a nitrogen-containing nonionic surfactant in an alcohol or solvent, an emulsion obtained by adding a nitrogen-containing nonionic surfactant to water and a water-miscible solvent followed by emulsifying them, and a solution obtained by dissolving a nitrogen-containing nonionic surfactant in a mineral oil, such as those in (1), (2) and (3) mentioned above, are useful as filters for air cleaners, absorbent cotton, wet tissues, sterilized non-woven fabrics, sterilized gauze, cleaning mops and dusters.

The present invention will be explained in more detail with reference to the following examples, in which parts and % are by weight.

[Example 1 and Comparative Example 1]

0.2 parts of polyoxyethylene oleic acid amide as a microbicide were added to 100 parts of middle or low-pressure polyethylene resin and filmed into a test film by injection molding (Example 1). A comparative film was made from only middle or low-pressure polyethylene in the same manner as above, without adding the microbicide thereto (Comparative Example 1).

A liquid containing about $10^4$ cells/ml of Escherichia coli or Staphylococcus aureus was dropped onto each test film, and a sterilized low-pressure polyethylene film was put thereover whereby the microbes-containing liquid was kept in close contact with the test film. This was stored at 25°C for 24 hours, and the number of living cells on the film was counted. The sterilized low-pressure polyethylene film was tested in the same manner as above, as a control film.

The test results are shown in Table 1.

[Table 1]

| Tested Microbes | Test Film | Time (hr) | |
|---|---|---|---|
| | | 0 | 24 |
| Escherichia Coli | Example 1 | | 10 or less |
| | Comparative Example 1 | $2.1 \times 10^4$ | $4.5 \times 10^5$ |
| | Control | | $1.3 \times 10^5$ |
| Staphylococcus aureus | Example 1 | | 10 or less |
| | Comparative Example 1 | $4.8 \times 10^4$ | $2.0 \times 10^3$ |
| | Control | | $1.8 \times 10^4$ |

[Example 2 and Comparative Example 2]

Polyoxyethylene linolamine as a microbicide was added to polypropylene resin in an amount of 0.2 % relative to the resin and the mixture was formed into a test plate according to the same process as in Example 1 (Sample A). Sample B was formed in the same manner as above, except that the amount of the microbicide was changed to 0.3 %. A comparative plate was formed without adding the microbicide thereto (Comparative Example 2).

These test plates were subjected to the same sterilizing test as that in Example 1. The sterilized low-pressure polyethylene film used as the cover film was also tested in the same manner as above as a control.

The test results are shown in Table 2.

[Table 2]

| Tested Microbes | Test Sample | Time (hr) | |
| --- | --- | --- | --- |
| | | 0 | 24 |
| Escherichia Coli | A | | 30 |
| | B | $2.1 \times 10^4$ | 30 |
| | Comparative Example 2 | | $4.6 \times 10^5$ |
| | Control | | $1.3 \times 10^5$ |
| Staphylococcus aureus | A | | 10 |
| | B | $4.8 \times 10^4$ | 10 or less |
| | Comparative Example 2 | | $8.2 \times 10^3$ |
| | Control | | $1.8 \times 10^4$ |

[Example 3]

Ethylamine oxide as a microbicide was dissolved in 70 parts of ethanol in an amount of 0.1 % (Sample A), 0.5 % (Sample B) and 1 % (Sample C) and heated at 40°C. While stirring, 30 parts of distilled water were added to each of them to prepare liquid microbicidal compositions.

One ml of a liquid containing microbes mentioned below (Table 3) was added to 10 ml of each sample composition and mixed and then reacted at 20°C for a determined period of time, 15 seconds, 30 seconds, one minute, 2 minutes and 5 minutes after the start of the reaction, the reaction mixture was sampled from each test tube and one platinum loop of the sample was transplanted into a culture medium. These were cultivated for 2 days at 35°C, whereupon the condition of the microbes in each culture medium was observed to check whether they died or were living.

As a control, sterilized water was tested in the same manner as above.

The test results are shown in Table 3, where "+" means that the microbes did not die and "-" means that the microbes died.

[Table 3]

| Tested Microbes | Tested Sample | Time | | | | |
|---|---|---|---|---|---|---|
| | | 15 sec | 30 sec | 1 min | 2 min | 5 min |
| _Escherichia coli_ | A | - | - | - | - | - |
| | B | - | - | - | - | - |
| | C | - | - | - | - | - |
| | Control | + | + | + | + | + |
| MRSA | A | - | - | - | - | - |
| | B | - | - | - | - | - |
| | C | - | - | - | - | - |
| | Control | + | + | + | + | + |

MRSA: Methicillin-resistant _Staphylococcus aureus_

[Example 4]

0.5 parts of a condensate composed of one mol of butylethanolamine and 20 mols of ethylene oxide, as a microbicide, were dissolved in 99.5 parts of ethanol, 100 parts of the resulting solution and 100 parts of LPG were formed into a sterilizing and deodorizing spray. This was sprayed over an agar plate medium (hereinafter referred to as a test plate), to which a liquid containing cells of MRSA, Micrococcus luteus, Staphylococcus aureus or Staphylococcus epidermidis had been applied, for one second, 3 seconds or 5 seconds, and then the cells were incubated on the test plate. After the incubation, the number of the colonies grown on the test plate was counted. A control test plate over which the test composition was not sprayed was also incubated in the same manner as above.

The test results are shown in Table 4 and Table 5.

EP 0 631 722 A1

[Table 4]

Results of Test for Microbicidal Activity against MRSA

| Spraying Time (sec) | Number of Colonies Grown on Test Plate | |
| --- | --- | --- |
| | Test 1 | Test 2 |
| 0 (control) | 142 | 167 |
| 1 | 0 | 1 |
| 3 | 0 | 0 |
| 5 | 0 | 0 |

[Table 5]

| Tested Microbes | Number of Colonies | | | |
| --- | --- | --- | --- | --- |
| | sprayed for 1 sec | sprayed for 3 sec | sprayed for 5 sec | control |
| Micrococcus luteus | 0 | 0 | 0 | 104 |
| Staphylococcus aureus | 0 | 0 | 0 | 221 |
| Staphylococcus epidermidis | 0 | 0 | 0 | 142 |

[Example 5]

0.3 parts of a condensate composed of one mol of ethylethanolamine monobietate and 5 mols of ethylene oxide as a microbicide were dissolved in 19.7 parts of ethanol, and the resulting solution was added to a solution prepared by dissolving 3 parts of an emulsifying agent (Pegunol #1500, made by Toho Chemical Co.) in 77 parts of distilled water under heat at 60°C, while stirring, to prepare a microbicidal emulsion containing the above-mentioned microbicide in water. Gauze was dipped in the resulting emulsion and this was used for wiping and cleaning desks, floors and passageways. The emulsion was excellent as a microbicidal emulsion for wiping and cleaning them.

The microbicidal emulsion was tested in the manner mentioned below to obtain the results shown in Table 6.

Object of Test:

The test is to examine the microbicidal activity of the emulsion against Methicillin-resistant Staphylococcus aureus (MRSA).

Summary of Test:

The emulsion was diluted to a 1/40 dilution (Sample 1) and a 1/50 dilution (Sample 2). Each dilution was poured into a laboratory dish and dried in air. A liquid containing microbes (MRSA) was dropped over each dish, whereupon the number of living cells was counted at regular intervals. The test results are shown in Table 6.

[Table 6]

Test Results

| Tested Microbes | Tested Sample | Number of Living Cells | | |
|---|---|---|---|---|
| | | Just after addition of cells(*1) | After 8 hours | After 24 hours |
| MRSA | Sample 1 | $2.8 \times 10^4$ | $<10$ | $<10$ |
| | Sample 2 | $2.8 \times 10^4$ | $<10$ | $<10$ |
| | Control(*2) | $2.8 \times 10^4$ | $1.6 \times 10^6$ | $1.6 \times 10^8$ |

(*1) The same microbes-containing liquid was used.
(*2) No microbicidal emulsion was added.

[Example 6]

A condensate composed of one mol of coconut oil fatty acid amide and 6 mols of ethylene oxide, as a microbicide, was kneaded with low-molecular paraffin wax in an amount of 1 % relative to the wax, and the resulting mixture was solidified into solid pellets. Several pellets thus formed were put in a vase with flowers, whereupon water in the vase was not putrefied. Thus, the pellets were effective in purifying water and in prolonging the life of the flowers in the vase.

**Claims**

1. A method for exterminating microbes for disinfection and deodorization, characterized by using a composition containing, as the active ingredient, a nitrogen-containing nonionic surfactant.

2. A method for exterminating microbes as claimed in claim 1, in which the nitrogen-containing nonionic surfactant is at least one selected from polyoxyethylene fatty acid amides, polyoxyethylene alkylamines, alkylamine oxides and fatty acid alkanolamide.

3. A method for exterminating microbes and for disinfection and deodorization as claimed in claim 1, in which the nitrogen-containing nonionic surfactant is at least one selected from polyoxyethylene fatty acid amides and polyoxyethylene alkylamines.

4. A method for exterminating microbes as claimed in claim 3, in which polyoxyethylene fatty acid amides are such that the fatty acid amide moiety in them has from 12 to 24 carbon atoms and the number of mols of ethylene oxide added to the moiety is 20 or less, and polyoxyethylene alkylamines are such that the higher alkylamine moiety in them has from 12 to 24 carbon atoms and the number of mols of ethylene oxide added to the moiety is 20 or less.

5. A method for exterminating microbes as claimed in claim 1, in which a solution composition or an emulsion composition containing, as the active ingredient, a nitrogen-containing nonionic surfactant is used.

6. A method for exterminating microbes as claimed in claim 5, in which a solution composition or an emulsion composition containing, as the active ingredient, a nitrogen-containing nonionic surfactant is directly sprinkled or sprayed.

7. A method for exterminating microbes as claimed in claim 5, in which a solution composition or an emulsion composition containing, as the active ingredient, a nitrogen-containing nonionic surfactant is sprayed over mops or dusters or mops or dusters are dipped in the composition.

8. A method for exterminating microbes as claimed in claim 5, in which a solution composition or an emulsion composition containing, as the active ingredient, a nitrogen-containing nonionic surfactant is sprayed over sanitary materials or sanitary articles or such materials or articles are dipped in the composition.

9. A method for exterminating microbes as claimed in claim 1, in which a wax containing a nitrogen-containing nonionic surfactant, as the active ingredient, added thereto by kneading is applied to floors.

10. A method for exterminating microbes as claimed in claim 1, in which oils and fats or waxes which are solid at a normal temperature or a crystalline resin containing a nitrogen-containing nonionic surfactant, as the active ingredient. added thereto by kneading is dipped in a liquid.

11. A method for exterminating microbes as claimed in claim 1, in which a thermoplastic resin containing a nitrogen-containing nonionic surfactant, as the active ingredient, is shaped into films, fibers and other shaped articles.

**European Patent Office**

# EUROPEAN SEARCH REPORT

Application Number

## DOCUMENTS CONSIDERED TO BE RELEVANT

EP 94401399.4

| Category | Citation of document with indication, where appropriate, of relevant passages | Relevant to claim | CLASSIFICATION OF THE APPLICATION (Int. Cl.5) |
|---|---|---|---|
| X | US - A - 3 916 003 (SUZUKI et al.) * Abstract * | 1-6 | A 01 N 25/30 A 61 L 2/16 |
| X | GB - A - 1 311 608 (AGENCY OF INDUSTRIAL SCIENCE & TECHNOLOGY) * Page 1, line 11 - page 2, line 11 * | 1-6 | |

TECHNICAL FIELDS SEARCHED (Int. Cl.5)

A 01 N
A 61 L

The present search report has been drawn up for all claims

| Place of search | Date of completion of the search | Examiner |
|---|---|---|
| VIENNA | 20-09-1994 | SCHNASS |